# EUROPEAN PATENT SPECIFICATION

(11) **EP 2 793 897 B1**
(45) Date of publication and mention of the grant of the patent: **12.04.2017**
(21) Application number: 12859788.7
(22) Date of filing: 14.12.2012
(51) Int. Cl.: A61K 31/575, A61P 17/08, A61P 17/10, A61P 17/18

(54) **TREATMENT OF SEBORRHOEA**
BEHANDLUNG VON SEBORRHOE
TRAITEMENT DE LA SÉBORRHÉE

(30) Priority: 20.12.2011 AU 2011905331; 22.02.2012 AU 2012900670
(43) Date of publication of application: 29.10.2014
(73) Proprietor: McFarlane Marketing (Aust.) Pty. Ltd., Abbotsford, Victoria 3067 (AU)
(72) Inventor: MACRIDES, Theodore, Lower Templestowe, Victoria 3107 (AU); BROADBENT, Andrew, Abbotsford, Victoria 3067 (AU)
(74) Representative: Dzieglewska, Hanna Eva
(86) International application number: PCT/AU2012/001543
(87) International publication number: WO 2013/090986

(56) References cited:
- WO-A1-95/10283
- JP-A- 2007 063 139
- JP-A- 2007 244 325
- US-A- 5 026 883
- MACRIDES T A ET AL: "COMPARISON OF THE HYDROXYL RADICAL SCAVENGING PROPERTIES OF THE SHARK RILE STEROID 5BETA-SCYMNOL AND PLANT PYCNOGENOLS", BIOCHEMISTRY AND MOLECULAR BIOLOGY INTERNATIONAL, ACADEMIC PRESS, LONDON, GB, vol. 42, no. 6, 1 September 1997 (1997-09-01), pages 1249-1260, XP000917911, ISSN: 1039-9712
- HARNEY ET AL: "Synthesis of an isomeric mixture (24RS,25RS) of sodium scymnol sulfate", STEROIDS, ELSEVIER SCIENCE PUBLISHERS, NEW YORK, NY, US, vol. 73, no. 4, 15 December 2007 (2007-12-15), pages 424-429, XP022493696, ISSN: 0039-128X, DOI: 10.1016/J.STEROIDS.2007.12.006

## Description

### FIELD OF THE INVENTION

The present invention relates to treatment of seborrhoea in a patient, and in particular relates to the use of scymnol, racemic at C24, and its esters (particularly its sulphate ester), in methods and compositions for the treatment of seborrhoea.

### BACKGROUND OF THE INVENTION

Seborrhoea (or hyperseborrhoea) is a medical condition characterised by excessive secretion by the sebaceous glands of sebum, an oily substance consisting mainly of fats which is produced by the disintegration of the cells in the sebaceous glands. The sebum reaches the skin surface through small ducts that lead from the sebaceous glands and open into the hair follicles. Some parts of the skin have many sebaceous glands, other parts have few such glands, and the activity of the sebaceous glands varies with age, the glands being most active at puberty.

In a patient with seborrhoea, the sebaceous glands are enlarged, especially beside the nose and other parts of the face. The condition predisposes to acne and is common at puberty, usually lasting for a few years.

Acne develops as a result of blockages in follicles. Hyperkeratinization and formation of a plug of keratin and sebum (a microcomedo) is the earliest change. The microcomedo may enlarge to form an open comedone (blackhead) or closed comedone (milia). Comedones are the direct result of sebaceous glands becoming clogged with sebum, a naturally occurring oil, and dead skin cells. In these conditions, the naturally occurring largely commensal bacterium *Propionibacterium acnes* can cause inflammation, leading to inflammatory lesions (papules, infected pustules, or nodules) in the dermis around the microcomedo or comedone, which results in redness and may result in scarring or hyperpigmentation.

The compound 24R-(+)-5β-cholestane-3α,7α,12α,24,26,27-hexol (designated as 24R-scymnol) occurs as a sulphate ester in fishes, rays and sharks, and is regarded as a typical component of the bile of all elasmobranch fish. Hammarsten (Z.Physiol.Chem. (1898) 24; 322) first described scymnol as an alcohol occurring as a sulphate in the bile of the northern shark *Scymnus borealis*.

The chemical structure of scymnol was reported by Bridgewater et al (Biochem. J. (1962) 82: 285) as 5β-cholestan-3α,7α,12α,24ε,26,27-hexol (or 5β-scymnol). However, the stereochemical configuration at the 24-position of scymnol was not identified by Bridgewater *et al -* there are three possibilities in the configuration at this position, namely 24R,24S or a racemic mixture of 24R and 24S. Bridgewater *et al* also disclosed a method for the isolation of scymnol from its naturally occurring sulphate, as well as a partial synthesis of scymnol (as a racemic mixture of the 24R and 24S compounds) from cholic acid. More recently, Ishida et al (Chem. Pharm. Bull. (Jpn), (1988) 36:4408) isolated, purified and examined both scymnol and its sulphate by nuclear magnetic resonance (n.m.r.) spectroscopy, and fully confirmed the structure of 24R-scymnol.

In prior International Patent Application No. PCT/AU87/00281 there is disclosed a process for the isolation and preparation of an active principle by extraction from particular tissues of sharks. This active principle, now termed "isolutrol", was isolated in good yield from an aqueous extract of the livers and/or gall bladders of sharks, and the active component therein identified as 24(+)-3α, 7α,12α,24,26-pentahydroxy-coprostane-27-sodium sulphate ester (sodium 24R-scymnol sulphate). This active component was also disclosed as being effective in the treatment of seborrhoea, particularly when used topically, for example in a topical cosmetic composition. Subsequently, it was disclosed in International Patent Application No. PCT/AU89/00064 that 24R-scymnol can be prepared from the active component disclosed in International Patent Application No. PCT/AU87/00281, and that 24R-scymnol has activity in the treatment of liver dysfunction.

In work leading to the present invention, it has now been found that scymnol, racemic at C24, (hereinafter referred to as (24RS)scymnol) and its esters, particularly its sulphate esters, and pharmaceutically acceptable salts of the esters, are effective in the treatment of seborrhoea.

### SUMMARY OF THE INVENTION

According to one aspect of the present invention, there is provided (24RS) scymnol, an ester thereof or a pharmaceutically acceptable salt of a said ester for use in a method for the treatment of seborrhoea in a patient which comprises administering to the patient an effective amount of (24RS) scymnol, an ester thereof or a pharmaceutically acceptable salt of a said ester.

Preferably, the administration is by the topical, dermal or transdermal route.

The present invention also extends to a composition for use in the treatment of seborrhoea in a patient, which comprises an effective amount of (24RS) scymnol or an ester thereof or a pharmaceutically acceptable salt of a said ester, together with a pharmaceutically acceptable carrier or diluent.

Preferably, the composition is a topical, dermal or transdermal composition.

In yet another aspect, the invention provides (24RS) scymnol or an ester thereof or a pharmaceutically acceptable salt of a said ester, for use in the treatment of seborrhoea in a patient.

Throughout this specification and the claims which follow, unless the context requires otherwise, the word "comprise", and or variations such as "comprises" or "comprising", will be understood to imply the inclusion of a stated integer or step or group of integers or steps but not the exclusion of any other integer or step or group of integers or steps.

### DETAILED DESCRIPTION OF THE INVENTION

The preparation of scymnol, racemic at C24, or (24RS) scymnol, using cholic acid as starting material is described by Amiet et al (Aust J. Chem (1993) 46; 1347-1354) and Harney and Macrides (Steroids (2008) 73: 424-429). Hamey and Macrides (2008) also describe the preparation of a sulphated (24RS) scymnol, which is monosulphated at C27, as a sodium salt, leading to a (24RS, 25RS) epimeric mixture of alcohols whereas the natural sodium scymnol sulphate purified from shark bile comprises the (24R, 25R) and/or (24R, 25S) epimers.

The structure of natural (24R,25RS) sodium scymnol sulphate is set out below:

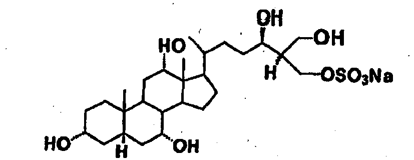

Also set out below are the structures of the four stereoisomers in the synthetic, racemic (24RS, 25RS) sodium scymnol sulphate prepared as described by Harney and Macrides (2008):

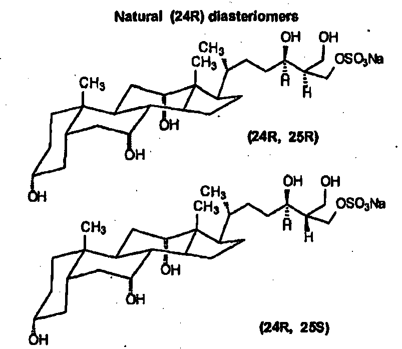

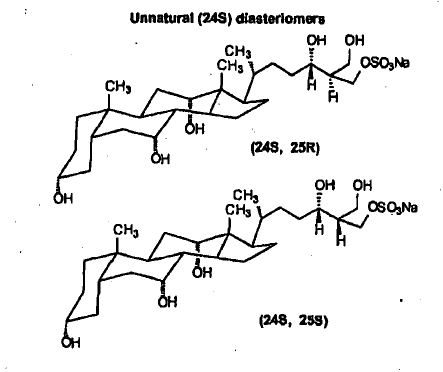

Enzymes and receptors in cells commonly select for one enantiomer of a chiral binding molecule (substrate or ligand) over the other enantiomer, because of the chirally-selective pockets inside their active sites. This is why most natural biological molecules (e.g. amino acids and sugars) are present in cells in only one chiral form.

Therefore, it is common for a specific enantiomer of a chiral biomolecule or drug to easily fit into the active site of a target enzyme or receptor with high binding affinity and strong bioactivity, whereas the other enantiomer either fits and binds differently, or doesn't fit at all. Thus, one drug enantiomer may produce a desired beneficial effect, while the other enantiomer may cause different beneficial effects and/or adverse effects. The presence of the non-beneficial enantiomer in a racemic mixture of chiral drug may result in a range of outcomes, e.g. halved beneficial bioactivity from the overall dose (if it is bioactivity (if it antagonises the beneficial enantiomer), or unwanted and/or adverse side effects (if it has a different bioactivity and/or toxicity profile to the beneficial enantiomer).

Given the common occurrence of chiral drugs and biomolecules having enantiomers with different bioactivities, a person skilled with this field would not assume that a racemic mixture and both of the individual enantiomers of a chiral bioactive molecule each have the same bioactivity profile, especially as such an assumption could be potentially dangerous. It is therefore necessary to investigate on a case-by-case basis and compare the bioactivity profile of a racemic mixture with its individual isomers, and elucidate their mechanisms of action, in order to accurately determine the overall bioactivity of a racemic mixture.

Consequently, the finding by the present inventors of the anti-sebum properties of racemic scymnol and scymnol sulphate, and the individual non-natural enantiomers of scymnol and scymnol sulphate is surprising, as these could not be assumed from the known anti-sebum properties of the natural scymnol and scymnol sulphate enantiomers.

According to one aspect of the present invention, there is provided (24RS) scymnol, an ester thereof or a pharmaceutically acceptable salt of a said ester for use in a method for the treatment of seborrhoea in a patient which comprises administering to the patient an effective amount of (24RS) scymnol, an ester thereof or a pharmaceutically acceptable salt of a said ester.

As disclosed above, the present invention also extends to a composition for use in the treatment of seborrhoea in a patient, which comprises an effective amount of (24RS) scymnol, an ester thereof or a pharmaceutically acceptable salt of a said ester, together with a pharmaceutically acceptable carrier or diluent.

In yet another aspect, the invention provides (24RS)-scymnol or an ester thereof or a pharmaceutically acceptable salt of a said ester, for use in the treatment of seborrhoea in a patient.

The esters of (24RS) scymnol which may be used in accordance with the present invention include esters with inorganic acids such as sulphuric acid or organic acids such as acetic acid, propionic acid or butyric acid. Where the ester is an ester with an inorganic acid such as sulphuric acid, it may be in the form of a pharmaceutically acceptable salt such as a sodium, potassium, calcium or ammonium salt, or an organic amine salt.

Preferably, the active substance is a sulphated form of (24RS) scymnol, more preferably the racemic (24RS,25RS) sodium scymnol sulphate prepared as disclosed by Harney and Macrides (2008) (hereinafter referred to as "synthetic racemic (24RS)-scymnol").

As used herein, references to treatment of seborrhoea include treatment of hyperseborrhoea or increased sebum production, as well as treatment of acne and other skin conditions which are connected with increased sebum production.

In accordance with this invention, the active substance will normally be administered dermally, transdermally or topically in the form of pharmaceutical preparations comprising an effective amount of the active substance in a pharmaceutically acceptable dosage form including a pharmaceutically acceptable carrier or diluent.

Suitable pharmaceutically acceptable dosage forms are well known and are described, by way of example, in Remington's Pharmaceutical Sciences, 18th Edition, Mack Publishing Company, Pennsylvania, USA. The dosage form may be a solid, semisolid or liquid preparation. Usually the active substance will constitute between 0.1 and 99% by weight of the preparation. Dermal, transdermal or topical administration would normally utilise 0.1-10% by weight, more specifically 0.5-5% by weight, of the active substance in a suitable dermal, transdermal or topical carrier or vehicle.

Preferably, the active substance is formulated so as to be administered topically or transdermally. In such formulations, an effective amount of the active substance is incorporated into a suitable carrier material as a topical pharmaceutical/cosmetic composition which may be made up in a variety of product types including, for example, lotions, creams, oils, gels, sticks, sprays, ointments, pastes, mousses and cosmetics.

Suitable topical pharmaceutical/cosmetic carrier materials for such compositions are also well known and are described, by way of example, in International Patent Application No. PCT/US91/02400. As described therein, in addition to the active substance and suitable carrier material, such topical pharmaceutical/cosmetic compositions may also include one or more penetration enhancing agent(s), and/or anti-inflammatory agent(s), as well as sunscreen or sunblock agent(s) to enhance protection of the skin against the effects of UV irradiation.

The compositions of the present invention may also incorporate known pharmaceuticals or other active ingredients, for example, antibiotics or other antibacterial substances. When formulated as a cosmetic composition, the active substance is formulated with a cosmetic base material, together with other cosmetic materials typically incorporated in cosmetic compositions.

The active substance is administered in the therapeutically effective amounts. A therapeutically effective amount means that amount necessary at least partly to attain the desired effect, or to delay the onset of, inhibit the progression of, or halt altogether, the onset or progression of the particular condition being treated. Such amounts will depend, of course, on the particular condition being treated, the severity of the condition and individual patient parameters including age, physical condition, size, weight and concurrent treatment. These factors are well known to those of ordinary skill in the art and can be addressed with no more than routine experimentation. It is preferred generally that a maximum dose be used, that is, the highest safe dose according to sound medical judgement. It will be understood by those of ordinary skill in the art, however, that a lower dose or tolerable dose may be administered for medical reasons, psychological reasons or for virtually any other reasons.

The following examples illustrate the use of active substances in accordance with this invention in methods and compositions for the treatment of seborrhoea.

### EXAMPLE 1 - Topical Compositions

The following compositions demonstrate typical compositions for topical use in the treatment of seborrhoea.

| | | |
|---|---|---|
| 1. | Cold cream | |
| | Spermacetti | 6.0 g |
| | Beeswax | 6.0 g |
| | Carbopol 934 | 10.0g |
| | Sodium Carbonate | 4.75 g |
| | Rose water | 5.0 ml |
| | Expressed almond oil | 56.0 g |
| | Active substance | 0.05 g |
| | Distilled water | 20.6 |
| 2. | Lotion | |
| | Ethanol | 30 ml |
| | Active substance | 20 mg |
| | Distilled water - sufficient quantity to make 100 ml | |

### EXAMPLE 2 - Clinical Studies

In an initial pilot study in humans, 3 topical formulations were evaluated for effectiveness in reduction of sebum or treatment of acne.

The active substance in each of these formulations was:
Formulation 1 - natural isolutrol (sodium 24R- scymnol sulphate).
Formulation 2 - synthetic (24R) - scymnol.
Formulation 3 - synthetic racemic (24RS) - scymnol.

In each formulation, the active substance was incorporated at 0.015% w/w into an oil-free topical lotion. In the pilot study, each test formulation was applied to one side of the face of a test subject, twice daily, for 28 days in the case of Formulations 1 and 2, and for 42 days in the case of Formulation 3. For each test formulation, 10 healthy female test subjects participated,in the pilot study.

For the evaluation of sebum reduction, a Sebumeter SM810PC (Courage + Khazaka electronic GmbH) was used to obtain measurement of skin sebum (skin surface lipids) on days 0, 3, 7, 14, 21, 28, 35 and 42. To accomplish this, a special purpose film of the cartridge measuring head was applied for 30 seconds to the relevant skin area. The cartridge was inserted into the Sebumeter SM810 PC for electronic determination of film transparency variations. The LC-display of the instrument presents the result in terms of µg/cm². Duplicated measurements were obtained on the same site at each visit, and the results were averaged.

For evaluation of anti-acne treatment, reduction in total acne lesion (pustules, papules and comedones) counts was measured.

The results of this initial study are shown in the accompanying figures, as follows:

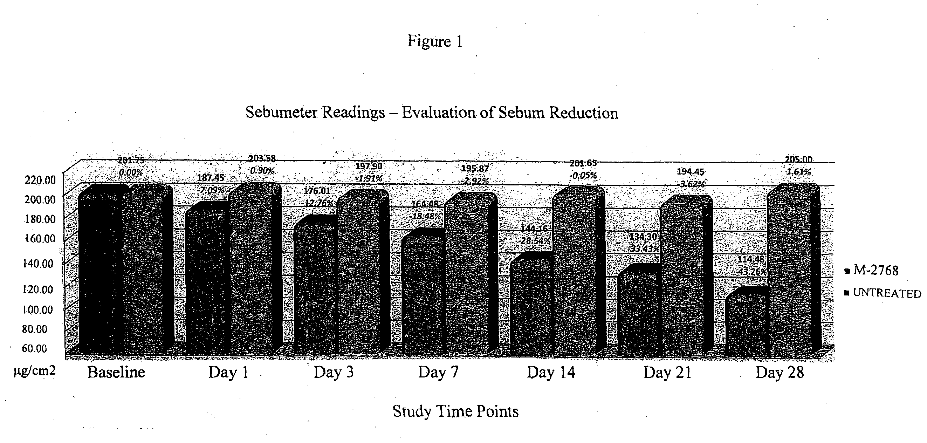

Figure 1- evaluation of sebum reduction using Formulation 1;

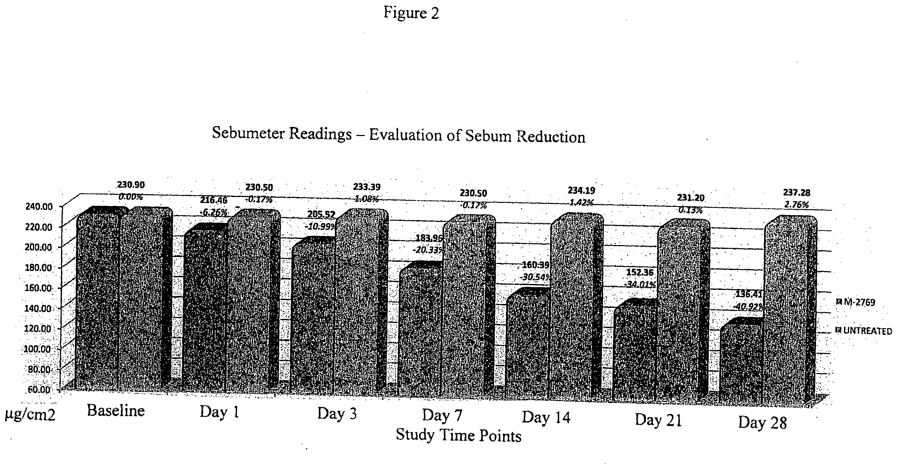

Figure 2 - evaluation of sebum reduction using Formulation 2;

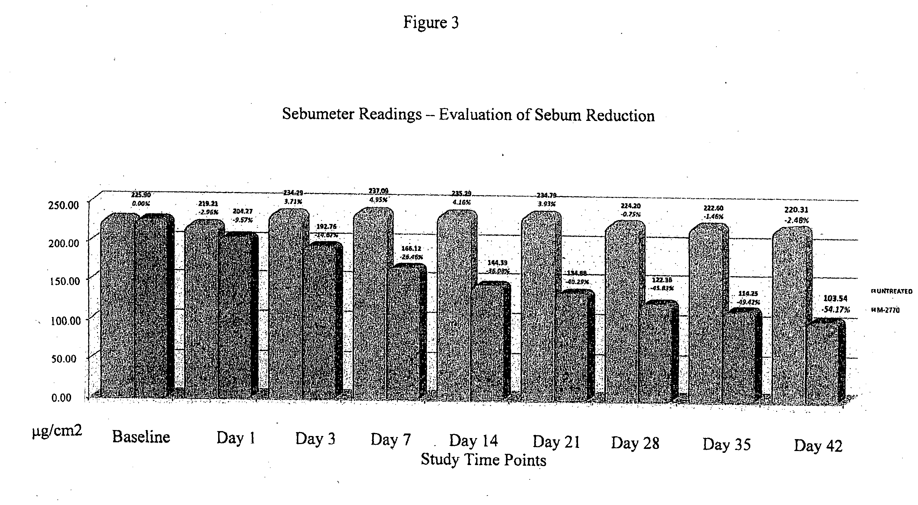

Figure 3 - evaluation of sebum reduction using Formulation 3;

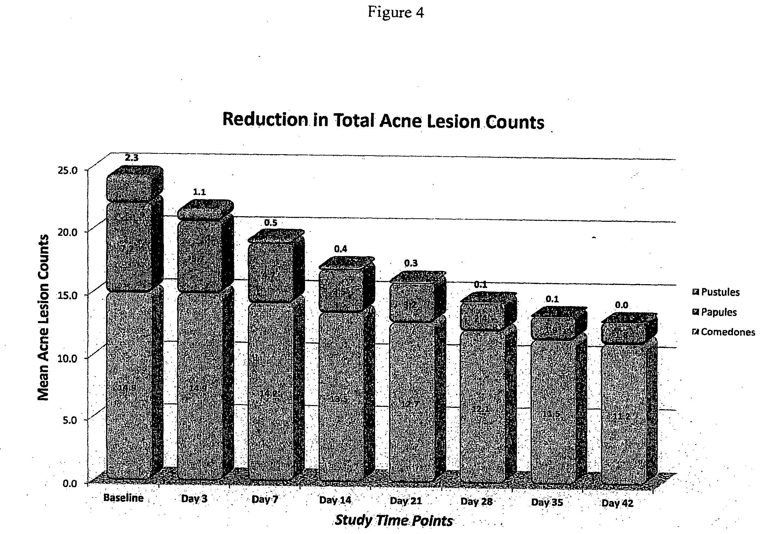

Figure 4 - reduction in total acne lesion counts using Formulation 3.

These results show that the active substance of Formulation 2, (24R)-scymnol, was surprisingly at least as effective in sebum reduction as the active substance of Formulation 1, natural sodium (24R)-scymnol sulphate after 28 days.

These results also show that the active substance of Formulation 3 used in both the sebum study and the acne study, racemic (24RS)-scymnol, was surprisingly at least as effective in sebum reduction as the active substances of Formulations 1 and 2, natural sodium (24R)-scymnol sulphate and (24R)-scymnol, respectively, after 28 days, and that sebum reduction continued when the test period was extended to 42 days. This active substance was also effective in reduction of total acne lesion counts over the 42 day test period. In particular, these results show that the presence of the non-natural (24S)-scymnol enantiomer in the racemic (24RS)-scymnol of Formulation 3 has no adverse effect in the sebum study.

## Claims

1. (24RS) scymnol, an ester thereof or a pharmaceutically acceptable salt of a said ester for use in the treatment of seborrhoea in a patient.

2. The (24RS) scymnol, ester or salt for use according to claim 1, wherein racemic (24RS, 25RS) sodium scymnol sulphate is used.

3. The (24RS) scymnol, ester or salt for use according to claim 1 or claim 2, wherein the treatment is by the topical, dermal or transdermal route.

4. A composition for use in the treatment of seborrhoea in a patient, which comprises an effective amount of (24RS) scymnol, an ester thereof or a pharmaceutically acceptable salt of a said ester, together with a pharmaceutically acceptable carrier or diluent.

5. The composition for use of claim 4, which comprises racemic (24RS, 25RS) sodium scymnol sulphate.

6. The composition for use of claim 4 or claim 5, which is a topical, dermal or transdermal composition.

## Patentansprüche

1. (24RS)-Scymnol, Ester davon oder pharmazeutisch akzeptables Salz eines der Ester zur Benutzung in der Behandlung von Seborrhö bei einem Patienten.

2. (24RS)-Scymnol, Ester oder Salz zur Benutzung nach Anspruch 1, wobei racemisches (24RS,25RS)-Natriumscymnolsulfat benutzt wird.

3. (24RS)-Scymnol, Ester oder Salz zur Benutzung nach Anspruch 1 oder 2, wobei die Behandlung auf dem topischen, dermalen oder transdermalen Weg erfolgt.

4. Zusammensetzung zur Benutzung in der Behandlung von Seborrhö bei einem Patienten, die eine wirksame Menge von (24RS)-Scymnol, eines Esters davon oder eines pharmazeutisch akzeptablen Salzes eines der Ester zusammen mit einem pharmazeutisch akzeptablen Träger oder Verdünnungsmittel umfasst.

5. Zusammensetzung zur Benutzung nach Anspruch 4, die racemisches (24RS,25RS)-Natriumscymnolsulfat umfasst.

6. Zusammensetzung zur Benutzung nach Anspruch 4 oder 5, die eine topische, dermale oder transdermale Zusammensetzung ist.

## Revendications

1. Scymnol (24RS), un ester de celui-ci ou un sel pharmaceutiquement acceptable d'un dit ester pour une utilisation dans le traitement de la séborrhée chez un patient.

2. Scymnol (24RS), ester ou sel pour utilisation selon la revendication 1, dans lequel du scymnol sulfate de sodium racémique (24RS, 25RS) est utilisé.

3. Scymnol (24RS), ester ou sel pour utilisation selon la revendication 1 ou la revendication 2, dans lequel le traitement est par voie topique, dermique ou transdermique.

4. Composition pour utilisation dans le traitement de la séborrhée chez un patient, qui comprend une quantité efficace de scymnol (24RS), d'un ester de celui-ci ou d'un sel pharmaceutiquement acceptable d'un dit ester, conjointement avec un support ou diluant pharmaceutiquement acceptable.

5. Composition pour utilisation selon la revendication 4, qui comprend du scymnol sulfate de sodium racémique (24RS, 25RS).

6. Composition pour utilisation selon la revendication 4 ou la revendication 5, qui est une composition topique, dermique ou transdermique.
